# EUROPEAN PATENT APPLICATION

(11) **EP 4 813 512 A1**
(43) Date of publication of application: **30.09.2026**
(21) Application number: 24892599.2
(22) Date of filing: 07.02.2024
(51) Int. Cl.: B01J 23/652

(54) **CATALYST FOR PREPARING ACRYLONITRILE BY PROPYLENE AMMOXIDATION, PREPARATION METHOD THEREFOR, AND USE THEREOF**

(30) Priority: 20.11.2023 CN 202311542085
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: LI, Jingxia, Shanghai 201208 (CN); ZHOU, Xiaofeng, Shanghai 201208 (CN); WU, Lianghua, Shanghai 201208 (CN)
(74) Representative: karo IP
(86) International application number: PCT/CN2024/076620
(87) International publication number: WO 2025/107431

(57) **Abstract**

The invention relates to a catalyst for preparing acrylonitrile by ammoxidation of propylene and a preparation method and a use thereof. The catalyst comprises active components and a carrier, wherein the active components comprise Mo, Bi, Fe, at least one rare earth element, at least one alkali metal element and at least one alkaline earth metal element; the catalyst has a molybdate crystal facet distribution index RQ of 0.65 to 0.98; wherein: RQ = (R' + Q')/(R + Q).

## Description

### Technical Field

The invention relates to the field of catalysis, and particularly relates to a catalyst with high stability for preparing acrylonitrile by ammoxidation of propylene, and to a preparation method and use thereof.

### Backgrounds

Acrylonitrile (AN) is a raw material monomer for synthesizing polyacrylonitrile fiber, is also a raw material for thermoplastic synthetic resins such as ABS, SAN and the like, nitrile rubber, adiponitrile, acrylamide and other derivatives, and is one of important petrochemical products. The technology for producing acrylonitrile by ammoxidation of propylene has become increasingly mature, and the development of an acrylonitrile catalyst with excellent performance is a hot point of attention in the acrylonitrile industry.

Currently, the industrial production of preparing unsaturated nitrile by olefin ammoxidation still commonly adopts a fluidized bed ammoxidation process. Catalysts, as one of the core technologies of this process, have always received significant attention for research and improvement. Currently, there are two main types of catalysts for the industrial preparation of acrylonitrile by ammoxidation of propylene: Mo-Bi serials and Sb serials, wherein Mo-Bi serial catalysts are dominant, accounting for 95% of olefin oxidation market. The previous researches and explorations are mainly focused on the Mo-Bi serial catalysts. The oxidation-reduction performance of the catalyst is improved by introducing metal components with variable valence states, such as Fe, Ce and other elements, into the catalyst, and the recovery of the effective state of active components of the catalyst is accelerated; the structure and stability properties of the catalyst can be improved by introducing metal elements with the ionic radius of more than 0.8nm and less than 0.8nm, such as Cr, Ni, Mg, Mn, Zn, Al and other elements, playing the role of structure and electronic assistant; the amount of lattice oxygen of the catalyst is increased and the catalytic performance of the catalyst is improved by introducing a rare earth element; the surface of the catalyst is modified and the acidity and basicity of the catalyst is adjusted by introducing elements such as Cs, Rb, P, B, Al and the like, improving the selectivity and the activity of the catalyst. It is stated in patent CN110557941A that the catalyst has a higher ammonia conversion and improves the yields of acrylonitrile and hydrogen cyanide as acrylonitrile ammoxidation products by controlling the composition and state of specific peaks in X-ray diffraction analysis. Further it is stated in patent CN113692315A that the yield of hydrogen cyanide can be increased while suppressing the decrease in the yield of acrylonitrile by controlling the composition of a specific phase in X-ray analysis. However, none of the above analyzes the phases of the catalyst, particularly a further analysis of the changes in phases closely associated with catalyst activity during the reaction. The catalysts in the prior art need to be improved in the aspects of long-term stable operation, high propylene conversion and the like. Therefore, it is necessary to analyze the phases of the catalyst and to prepare a catalyst exhibiting high propylene conversion during long-term operation.

### Disclosure of Invention

In view of the defects in the prior art, the invention aims to provide a catalyst with high stability for preparing acrylonitrile by ammoxidation of propylene, which catalyst exhibits the advantages of high propylene conversion in the long-term operation process of the catalyst.

In order to realize the above purpose, the invention provides the following technical solution:
a catalyst, preferably a catalyst for preparing acrylonitrile by ammoxidation of propylene, the catalyst comprises active components and a carrier; the active components comprise Mo, Bi, Fe, at least one rare earth element, at least one alkali metal element and at least one alkaline earth metal element; the catalyst has a molybdate crystal facet distribution index RQ of 0.65 to 0.98, preferably 0.70 to 0.98; wherein: $RQ = \left(R′+Q′\right) / \left(R+Q\right) ,$
wherein the R and Q are respectively determined as follows:
a certain amount of catalyst is taken, and the peak areas of peaks at 20 positions of 22.8°±0.2 (R) and 25.5°±0.2 (Q) in the X-ray diffraction of the catalyst are determined, and
the R' and Q' are respectively determined as follows:
   under the condition without oxygen, a certain amount of catalyst is taken, and propylene and nitrogen are introduced, wherein the gas molar ratio of the propylene to the nitrogen is 0.25, the reaction temperature is 430 °C, the catalyst weight space velocity is 0.18h⁻¹, and the residence time is 0.3s, so that one pulse-reduction is completed; the pulse-reduction is repeatedly implemented after an interval of 10min; and after implementing the pulse-reduction for 10 times, the peak areas of peaks at 20 positions of 22.8°±0.2 (R') and 25.5°±0.2 (Q') in the X-ray diffraction of the catalyst after 10 times of pulse-reduction are determined.

In practical operations, to determine the R and Q, and R' and Q', respectively, preferably, a certain amount (for example 1-5 g) of catalyst is taken and is divided on average into two portions, one for the determination of the R and Q and the other for the determination of the R' and Q'.

It will be understood by those skilled in the art that for the purposes of the present invention, when it is stated that a "peak" is at a certain 20 position, it is actually meant that the summit of the peak is at that position.

The catalyst of the invention exhibits a structure of Fe element enrichment in its bulk, wherein the Fe element has a Fe distribution index D_{Fe} between bulk and surface of 10-40%, wherein: ${D}_{Fe} = \left({C}_{total Fe}-{C}_{surface Fe}\right) / {C}_{total Fe} ;$
wherein the C_{total Fe} represents the total Fe content of the whole catalyst;
the C_{surface Fe} represents the Fe content on the catalyst surface.

The catalyst of present invention has a molybdate phase ratio index T/R of 2 to 5, wherein T is the peak area of peak at 20 position of near 28.2° ± 0.2 in the X-ray diffraction of the catalyst.

The present invention also provides a method for preparing the catalyst of the present invention, wherein, the amounts of the active components including Mo and the carrier are determined, and comprises the following steps performed in sequence:
(1) forming solutions respectively by adding each of the entire amount of the source of Fe element, the entire amount of the source of the at least one rare earth element and a partial amount of the source of Mo element in the active components into a solvent, mixing the solutions and adding in a dispersant to form a mixed liquid I, and subjecting the mixed liquid I to a heat treatment;
(2) forming a solution by adding the remaining amount of the source of Mo element into a solvent, and mixing the solution with the entire amount of the source of the carrier to form a mixed liquid II;
(3) mixing the sources of other active components besides the active components used in steps (1) and (2) in entire amounts and adding into a solvent to form a solution III;
(4) adding the mixed liquid I into the mixed liquid II and mixing to form a mixed liquid IV;
(5) adding the solution III into the mixed liquid IV to form a slurry V;
(6) heat treating the slurry V and drying to obtain a particulate matter;
(7) optionally, calcining the particulate matter to obtain the catalyst in particle form;
wherein, the partial amount of the source of Mo element added in the step (1) accounts for 40-80% of the entire source of Mo element.

### Description of Drawings

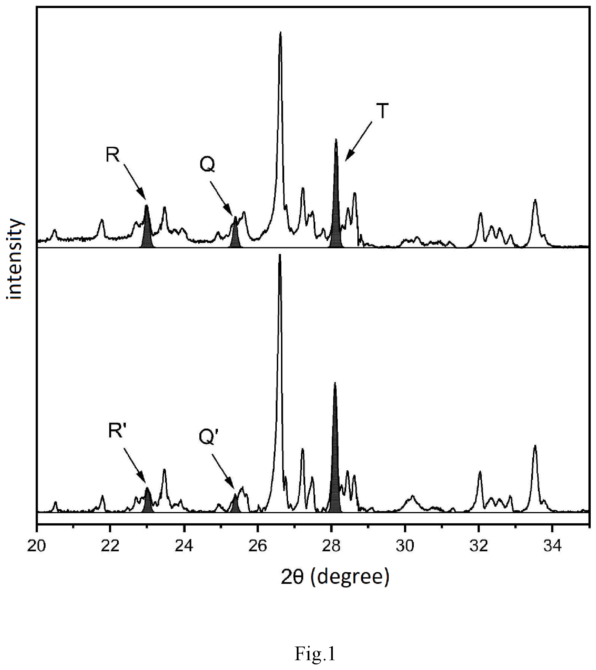

Figure 1 shows the XRD pattern for measuring R and Q, and R' and Q' for the catalyst of Example 1.

### Detailed Description

The endpoints of the ranges and any values disclosed in the present application are not limited to the precise ranges or values and should be understood to encompass values close to these ranges or values. For numerical ranges, one or more new numerical ranges can be obtained by arbitrarily combinations between the endpoint values of the ranges, the endpoint values and specific point values within the ranges, and the specific point values themselves, and such numerical ranges should be construed as specifically disclosed herein.

In the present invention, unless otherwise specifically mentioned, the description of amounts, contents and related ratios thereof is made on a "weight" basis, unless it is obvious that it is not in accordance with the conventional understanding in the art.

In the present invention, unless otherwise indicated, the terms "comprising," "including," "containing," "having," and similar words are to be construed as open-ended descriptions, but also construed to mean that closed-ended descriptions are explicitly disclosed at the same time. For example, "comprising" means that it may include other elements not listed, but also explicitly disclosed as including only the listed elements. Furthermore, as used herein, "comprising/including" is interpreted as specifying the presence of the stated features, integers, steps or components as referred to, but does not preclude the presence or addition of one or more other features, integers, steps, components or groups thereof. Additionally, the term "comprising" is intended to include embodiments encompassed by the terms "consisting essentially of ..." and "consisting of ...". Similarly, the term "consisting essentially of ..." is intended to include embodiments encompassed by the term "consisting of ...".

In the present invention, the active components and the carrier of the catalyst each have a meaning known in the art. For example, an active component is understood to be the form in which an active element playing a catalytic role (including a cocatalytic role) in the catalyst is present, and the carrier is the component which serves to carrier the active component and which, for example, can be inert to the reaction. It will be appreciated by those skilled in the art that, for convenience of description, the term "active component" may also be used herein to describe the active element itself, as appropriate.

The invention provides a catalyst, preferably a catalyst for preparing acrylonitrile by ammoxidation of propylene, the catalyst comprises active components and a carrier; the active components comprise Mo, Bi, Fe, at least one rare earth element, at least one alkali metal element and at least one alkaline earth metal element; the catalyst has a molybdate crystal facet distribution index RQ of 0.65 to 0.98, preferably 0.70 to 0.98; wherein: $RQ = \left(R′+Q′\right) / \left(R+Q\right) ,$ wherein the R and Q are respectively determined as follows:
a certain amount of catalyst is taken, and the peak areas of peaks at 20 positions of 22.8°±0.2 (R) and 25.5°±0.2 (Q) in the X-ray diffraction of the catalyst are determined, and
the R' and Q' are respectively determined as follows:
   under the condition without oxygen, a certain amount of catalyst is taken, and propylene and nitrogen are introduced, wherein the gas molar ratio of the propylene to the nitrogen is 0.25, the reaction temperature is 430 °C, the catalyst weight space velocity is 0.18h⁻¹, and the residence time is 0.3s, so that one pulse-reduction is completed; the pulse-reduction is repeatedly implemented after an interval of 10min; and after implementing the pulse-reduction for 10 times, the peak areas of peaks at 20 positions of 22.8°±0.2 (R') and 25.5°±0.2 (Q') in the X-ray diffraction of the catalyst after 10 times of pulse-reduction are determined.

The invention provides the above specific determination method for R' and Q'; however, it will be appreciated by those skilled in the art that the specific determination method is exemplary and that the determination method may be reasonably flexible so that consistent or substantially consistent determination results can be obtained over certain ranges of measuring conditions. For example, a certain amount of catalyst can be taken, and, under the condition without oxygen, propylene and nitrogen are introduced, wherein the gas molar ratio of the propylene to the nitrogen is 0.20-0.30, the reaction temperature is 430-440 °C, the catalyst weight space velocity is 0.15-0.20h⁻¹, and the residence time is 0.2-0.5s, so that one pulse-reduction is completed; the pulse-reduction is repeatedly implemented after an interval of 5-15min; and after implementing the pulse-reduction for 10 times, the peak areas of peaks at 20 positions of 22.8°±0.2 (R') and 25.5°±0.2 (Q') in the X-ray diffraction of the catalyst after 10 times of pulse-reduction are determined.

The peak area described in this embodiment has a meaning known in the art and can be determined by a person skilled in the art based on knowledge known in the art and by known techniques; for example, the peak area can be the area of a region surrounded by a Gaussian function curve having a mean value within a specified 20 angle range of and the base line, after peak splitting and fitting of a spectrum within a range of 2θ angle ± 1° defined by XRD diffraction pattern by using a Gaussian function.

The baseline is a line formed by connecting peakless points in the XRD diffraction pattern.

It should be noted that, the above "peakless point" refers to a point on the so-called baseline in a graph in which the vertical axis represents the diffraction X-ray intensity and the horizontal axis represents 20 in the XRD measurement, and indicates a point where no peak is present. Since XRD measurement usually will include a certain level of noise and the like, a certain level of diffracted X-ray intensity will be exhibited even at a position having no peak (no diffraction point), and for this purpose, a line with almost zero diffracted X-ray intensity is mechanically obtained by calculation based on the measuring instrument, that is, by calculating noise and background according to the overall analysis results, and this line is taken as the baseline.

In addition, in the case where the baseline is significantly inclined with respect to the X-axis representing 20, the graph may be corrected as appropriate using the analysis software accompanying the XRD apparatus used in the measurement so that the baseline is parallel to the X-axis, and then the peak area can be calculated.

The peak splitting and fitting refers to a process of performing peak identification on a spectral curve obtained by XRD measurement and performing curve fitting through the least square method by using Gaussian function, using the analysis software accompanying the XRD apparatus or other data processing software capable of conducting curve fitting.

The catalyst of present invention exhibits a structure of Fe element enrichment in its bulk, wherein the Fe element has a Fe distribution index D_{Fe} between bulk and surface of 10-40%, wherein: ${D}_{Fe} = \left({C}_{total Fe}-{C}_{surface Fe}\right) / {C}_{total Fe} ;$
wherein the C_{total Fe} represents the total Fe content of the whole catalyst;
the C_{surface Fe} represents the Fe content on the catalyst surface.

In the context of the present invention, the "surface Fe element content (C_{surface Fe})" of the catalyst particles is determined using X-ray photoelectron spectroscopy (XPS). Here, the "surface" means the range of the depth of the catalyst surface that can be measured by XPS (generally <10 nm). By way of example, in the context of the present invention, the equipment used for performing XPS measurements can be a model AXIS Ultra DLD X-ray photoelectron spectrometer from Kratos Analytical Ltd; in the sample preparation stage, the catalyst powder is directly fixed (for example, by using a double-sided adhesive tape) onto a copper sheet and then the copper sheet is placed on the sample table; before testing, the vacuum in the sample analysis chamber is ensured to be better than 1.0 x 10⁻⁸ mbar before turning on the X-ray source. As an example, the specific testing steps can be: roughly adjusting the position of the sample by using CCTV, and adjusting the testing position to the position which is most clear in imaging and is positioned at the central marked position of the imaging position; exciting the light source by Mg Kα, and pre-scanning the full spectrum and determining the contents, the positions and the strongest peak position of the sample elements by using the current of 40 mA; adjusting the height of the sample to the strongest signal at the position of the strongest peak; collecting and storing sample spectrogram information; after obtaining XPS spectral line, correcting by C 1s spectral line of contaminating carbon; from these peak area proportions, the mole% of surface Fe element is calculated as C_{surface Fe} for the catalyst particles.

In the context of the present invention, the "total Fe element content (C_{total Fe})" of the catalyst particles is determined using X-ray fluorescence analysis. By way of example, the total Fe element content of the catalyst can be determined herein by using an S4 Pioneer X-ray fluorescence spectrometer of Bruker, Germany. As an example, in the sample preparation stage, 3g of catalyst and 3g of boric acid are mixed, then are subjected to ball milling in a ball mill for 1.5min, and then are pressed into a tablet; the mole% of Fe element in the sample is determined, as C_{total Fe} of the catalyst particles, by measuring the intensity of the secondary X-ray generated by the sample and comparing it with the intensity of the secondary X-ray generated by the standard sample using X-ray as the excitation light source.

Accordingly, in the context of the present invention, when reference is made to the distribution of an element or component in the catalyst, for the purposes of the present invention, the "bulk" refers to the portion of the catalyst as a whole other than the "surface".

In the context of the present invention, the phases of the catalyst particles are determined using an X-ray powder diffractometer. By way of example, the catalyst phases can be determined herein using a D8 AdvanceSS X-ray powder diffractometer from Bruker, Germany; a Cu target is used, the light source wavelength is 0.6887 Å, room temperature and atmospheric pressure, and the sample is ground to 200 mesh as powder and placed in a sample cell for measurement.

Further, the catalyst of present invention has a molybdate phase ratio index T/R of 2 to 5, wherein T is the peak area of peak at 20 position of near 28.2 ° ± 0.2 in the X-ray diffraction of the catalyst.

It is readily and well understood by those skilled in the art that a substance is generally determined by both components and structures, either of which may cause a great change in the physicochemical properties of the substance. In particular, in the case of a catalyst, the structure thereof in addition to the catalyst components greatly affects various properties of the catalyst. For example, for multi-component catalysts, there are a variety of structures commonly used in the art, such as blending, surface coating, pore loading, bulk-enriched structure employed in the present invention, and core-shell structure; even with the core-shell structure, there is a problem of how the individual components are distributed in the core and shell.

The invention aims to provide a catalyst for preparing acrylonitrile by ammoxidation of propylene. For such catalysts, it is well known to those skilled in the art that they are subjected to reducing conditions during the reaction; therefore, such catalysts preferably have good anti-reduction properties, thereby exhibiting high stability during long-term operation of the catalysts. For example, for a catalyst with given components, a simple method for the component which may be reduced can be encapsulate it, either completely or partially; however, it is readily understood that such encapsulation may not sufficiently expose the active sites, resulting in an undesirable reduction in catalytic activity. Therefore, the art seeks to impart desirable anti-reduction ability to the catalyst while trying to obtain high catalytic performance by an appropriate structure.

The catalyst of the invention comprises active elements such as Mo, Bi, Fe and the like, and as known in the art, such a catalyst for preparing acrylonitrile by ammoxidation of propylene generally forms molybdates, such as iron molybdate during the preparation process. The inventors of the invention have unexpectedly found that a proper distribution of the active elements such as Mo, Bi and/or Fe and the corresponding molybdates formed in the bulk and the surface of the catalyst to obtain a bulk-enriched structure, is particularly beneficial to impart the catalyst with desirable anti-reduction capability while obtaining high catalytic performance as much as possible. It is known in the art that such catalysts for preparing acrylonitrile by ammoxidation of propylene contain a crystalline structure, and lattice oxygen plays an important role in the catalytic process.

Without being bound by any known theory, it is believed that when the specific catalyst components selected by the present invention are used, by using the specific preparation steps described in the present invention, especially, for example, adding the Mo element source in steps rather than in one time, mixing and treating specific components in each step, and using specific mixing order among the mixtures obtained in each step, etc., optionally in combination with specific treating conditions, the active Fe element is mainly concentrated in the inside of the crystal, and specific crystal facets, crystal phase compositions and distributions are obtained, which are beneficial for imparting a desirable anti-reduction ability to the catalyst while attaining as high catalytic performance as possible. Without being bound to any known theory, it is believed that the structures such as specific element distribution, crystal facet and crystal phase distribution, etc. of the catalyst according to the present invention prepared by a specific method can be represented by the molybdate crystal facet distribution index RQ, the molybdate phase ratio index T/R, etc., after in-depth research and experimental verification.

It is understood by those skilled in the art that the present invention produces a catalyst having a specific structure by the specific method; however, one skilled in the art can adjust the steps or its order, conditions and/or raw materials, etc. of the method based on the specific situation and expertise, so long as the catalyst having the molybdate crystal facet distribution index RQ, the molybdate phase ratio index T/R, etc. can be obtained.

For the present invention, the active Fe element mainly concentrated inside the crystal can be represented by Fe distribution index D_{Fe}: ${D}_{Fe} = \left({C}_{total Fe}-{C}_{surface Fe}\right) / {C}_{total Fe} ;$ wherein the total Fe content in the bulk of catalyst represented by C_{total Fe} is the total Fe content of the whole catalyst measured by the X-ray fluorescence analysis method; and the Fe content on the surface of the catalyst represented by C_{surface Fe} is the Fe content measured by the XPS analysis. It can be understood by those skilled in the art that XPS analysis can be used to characterize the Fe content on the surface of the tested object according to the principle and testing method of XPS analysis; both C_{total Fe} and C_{surface Fe} are based on the total weight of the tested subject catalyst.

The catalyst of present invention is subjected to reduction treatment, and the ratio of the sum of peak areas of at 2θ positions near 22.8°±0.2 and 25.5°±0.2 in X-ray diffraction before and after the reduction is examined, so that the influence rule of the composition change of the key active phase in the catalyst in the reduction process to the activity and stability of the catalyst is obtained; the inventors have found during research that the catalyst of the present invention can be stably operated for a long period of time and can provide a high conversion of propylene in the reaction for producing acrylonitrile by ammoxidation of propylene when its (R' + Q')/(R + Q) is in the range of 0.65 to 0.98, preferably 0.70 to 0.98.

The reduction treatment conditions of the above mentioned catalyst are as follows: under the oxygen-free condition, carrying out in-situ pulse-reduction of the catalyst in propylene and nitrogen atmosphere;
preferably, the conditions for in-situ pulse-reduction are as follows:
under the condition without oxygen, taking 1g of catalyst and carrying out pulse-reduction by introducing propylene and nitrogen, wherein the gas molar ratio of the propylene to the nitrogen is 0.25, the reaction temperature is 430 °C, the catalyst load (weight space velocity) is 0.18h⁻¹, and the residence time is 0.3s; with an interval of 10min, the pulse-reduction being repeated; and after implementing the pulse-reduction for 10 times, a catalyst sample after pulse-reduction being obtained.

The pulse-reduction method may be reasonably flexible so that consistent or substantially consistent determination results can be obtained over certain ranges of measuring conditions. For example, a certain amount of catalyst can be taken, and, under the condition without oxygen, propylene and nitrogen are introduced, wherein the gas molar ratio of the propylene to the nitrogen is 0.20-0.30, the reaction temperature is 430-440 °C, the catalyst weight space velocity is 0.15-0.20h⁻¹, and the residence time is 0.2-0.5s, so that one pulse-reduction is completed; the pulse-reduction is repeatedly implemented after an interval of 5-15min; and the pulse-reduction is implemented for 10 times in total.

In a preferred technical solution of the present invention, the rare earth element is at least one selected from La, Ce, Pr, Nd, and Sm, preferably at least one of La, Ce, and Nd; and/or,
the alkali metal element is at least one selected from Li, Na, K, Rb and Cs, preferably K and Rb; and/or,
the alkaline earth metal element is at least one selected from Be, Mg, Ca, Sr and Ba, and is preferably Mg and Ca; and/or,
the element A is at least one selected from W, V, Zr, P, Nb, Ni, Co, Cr, Mn, Tl, Au, Ag, Pt, Ru, Rh, Pd, Ti, Sb, In, Sn and Te, preferably at least one selected from W, Zr, P, Nb, Ni, Co, Cr, Ag, Mn and In.

The carrier used in the present invention can be one commonly used in the art, and is preferably a neutral or substantially neutral carrier. Accordingly, it is preferred that the carrier is silica.

In a preferable technical solution of the invention, the content of the active components is 30-90%, preferably 50-70%, calculated by oxide, based on the weight of the catalyst; the content of the carrier is 10-70%, preferably 30-50%.

Further, in the above technical solutions, in the active components, based on the weight of the catalyst particles:
the weight content of the Mo element is 15-55%, preferably 20-45%, calculated as MoO₃;
the weight content of the Bi element is 0.5%-3.5%, preferably 1.0%-3.5%, calculated as Bi₂O₃;
the weight content of the Fe element is 1%-12%, preferably 1.5%-11%, calculated as Fe₂O₃;
the weight content of the rare earth element is 1.5%-8.5%, preferably 2.5%-5.0%, calculated as the oxide of the rare earth element;
the weight content of the alkali metal element is 0.01%-0.60%, preferably 0.05%-0.55%, calculated as oxide;
the weight content of the alkaline earth metal is 0.01%-4.0%, preferably 0.5%-3.5%, calculated as oxide;
the weight content of the element A is 0.01%-15%, preferably 0.05%-14%, calculated as oxide

In a preferred technical solution of the present invention, in the active components:
the Bi/Mo atomic ratio is 0.008-0.25, preferably 0.01-0.20;
the Fe/Bi atomic ratio is 1.0-12.0, preferably 1.5-11.0;
the atomic ratio of the sum of the rare earth element, the alkali metal element and the alkaline earth metal element to Mo is 0.05-0.4, preferably 0.10-0.35; and/or,
the atomic ratio of element A/Mo is 0.01 to 1.0, preferably 0.02 to 0.9. The above specified ratio ranges are beneficial for the formation of a phase having better catalyst activity in the catalyst.

Another object of the present invention is to provide a method for preparing a catalyst with high stability for producing acrylonitrile by ammoxidation of propylene, which comprises the following steps performed in sequence:
(1) forming solutions respectively by adding each of the entire amount of the source of Fe element, the entire amount of the source of the at least one rare earth element, and a partial amount of the source of Mo in the active components into a solvent, mixing the solutions and adding in a dispersant to form a mixed liquid I, and subjecting the mixed liquid I to a heat treatment;
(2) forming a solution by adding the remaining amount of the source of Mo element into a solvent, and mixing the solution with the entire amount of the source of the carrier to form a mixed liquid II;
(3) mixing the sources of other active components besides the active components used in steps (1) and (2) in entire amounts and adding into a solvent to form a solution III;
(4) adding the mixed liquid I into the mixed liquid II and mixing to form a mixed liquid IV;
(5) adding the solution III into the mixed liquid IV to form a slurry V;
(6) heat treating the slurry V and drying to obtain a particulate matter;
(7) optionally, calcining the particulate matter to obtain the catalyst in particle form;
wherein, the partial amount of the source of Mo element added in the step (1) accounts for 40-80% of the entire source of Mo element.

It is understood that, although the steps involved in the method of the invention being "performed in sequence" is an important feature for the purposes of the invention, it is emphasized primarily those steps in the method which are clearly in a sequential process relationship; for example, step (4) requires addition of mixed liquid I into mixed liquid II, and thus step (4) should not and cannot be performed prior to step (1) of forming mixed liquid I or prior to step (2) of forming mixed liquid II. However, for some steps with no obvious sequential process relationship, the skilled person can adjust or exchange the order of the steps as appropriate, for example, based on the steps (1) - (7) described above:
- the order may be exchanged arbitrarily between steps (1), (2) and (3), to form, for example, the orders of: steps (1), (3) and (2); steps (2), (1) and (3); steps (2), (3) and (1); steps (3), (1) and (2); or steps (3), (2) and (1);
- the order between steps (3) and (4) can be exchanged to form the order of steps (1), (2), (4) and (3); and
- the order may be exchanged between steps (1) and (2) and the order may be exchanged between steps (3) and (4) to form the order of steps (2), (1), (4) and (3).

In the above discussion of the adjustment or exchange of the order of steps, a step not specifically mentioned means its original order of steps remains unchanged.

It is understood that in the present invention, the "source" of various elements/components has the meaning known in the art, which for example means various substances added as starting materials for the introduction of the various active components/elements in the catalyst of the invention. It is understood by those skilled in the art that the form of the objective element/component to be introduced in the source may be different from that in the catalyst. For example, the carrier of the catalyst of the invention may preferably be in the form of silica, while the source thereof may preferably be in the form of a silica sol.

It is understood that, in the present invention, "soluble" is mentioned with respect to the respective corresponding solvent. In the present invention, there is no particular limitation on the types of solvents, and conventional solvents in the art can all be used in the present invention. For example, the solvent of the present invention may be independently at least one selected from water, methanol, ethanol, and ethyl acetate, and is preferably water.

In the steps (1) and (2), the order of adding and mixing the various materials is not particularly limited as long as the mixed liquids I and II can be formed, respectively. However, without being bound to any known theory, it is believed that the manner in which mixed liquid I is added into mixed liquid II for mixing in step (4) is particularly advantageous for obtaining the catalyst particles required in the present invention, compared to other mixing manners, such as adding mixed liquid II to mixed liquid I. For this purpose, it is preferable to control the rate of addition of mixed liquid I into mixed liquid II, for example, adding mixed liquid I into mixed liquid II at a constant rate in 5-15 min. Similarly, in the step (5), the manner of adding the solution III into the mixed liquid IV is particularly advantageous for obtaining the catalyst particles required in the present invention. For this purpose, it is preferable to control the rate of addition of mixed liquid III into mixed liquid IV, for example, adding mixed liquid III into mixed liquid IV at a constant rate in 5-15 min.

The method can further stabilize the active phase in the reaction process. As mentioned above, the source of Mo element is added in steps rather than in one time, specific components are mixed and treated in each step, and the mixtures obtained in the steps have a specific mixing sequence, so that in the process of coprecipitation, a mixed liquid is formed firstly by the sources of Fe and at least one rare earth element such as La, Ce and the like and part of source of Mo element, and an eutectic structure with a stable active phase can be formed in the mixed liquid by adding a dispersant and optionally carrying out heat treatment, which is then added into the mixed liquid formed by other elements and the remaining amount of source of Mo element; the above treatment and precipitation processes facilitate the formation of a uniform active phase in the catalyst and improve the stability of the highly active phase, helping the catalyst to maintain good stability during the reduction process of the reaction.

In the above technical solutions, a source of the active component is a compound containing an element of the active component, and the compound is preferably a water-soluble compound, and more preferably a water-soluble salt; such as nitrates, sulfates, hydrochlorides, oxalates, and the like.

In the above technical solutions, the solvent of the mixed liquid and/or solution can preferably be water. The concentration of the mixed liquid and/or solution may not be particularly limited as long as the source(s) of the active component(s) and the source of carrier can be sufficiently mixed and dissolved. Preferably, for the present invention, the solutions formed by the sources of the various active components are each saturated solutions of the sources of the active components. Without being bound by any known theory, it is believed that carrying out the present invention with saturated solutions of the sources of various active component is particularly advantageous for obtaining the catalyst particles required by the present invention, especially for example, obtaining a desirable distribution of various elements/components.

As mentioned above, the addition of the source of Mo element in steps rather than all at once is particularly advantageous for forming the desired catalyst of the present invention. Accordingly, in a preferred technical solution of the present invention, the amount of the source of Mo element added in the step (1) is 40 to 80%, preferably 50 to 75% of the total source of Mo element.

As mentioned above, the mixing and treating of specific components in each step and the specific mixing sequence of the mixtures obtained in the steps in the present invention is particularly advantageous for forming the desired catalyst of the present invention. Accordingly, in steps (1) and (2) of the method of the present invention, solutions of the sources of the various elements/components are first formed respectively and then mixed to form the mixed liquid I. In contrast, for step (1) or (2), the present invention does not recommend mixing the sources of two or more elements/components and then forming a solution; it is also not recommended, for step (1) or (2), to form a solution of a source of an element/component and then adding sources of other elements/components thereto.

As mentioned above, the present invention is particularly advantageous for forming the desired catalyst according to the present invention by using specific treatment conditions. Wherein, for example, with respect to step (1), as described above, there can be no particular limitations to the amount of the solvent added and thus the concentration of the mixed liquid I; thus, in the step (1), the dispersibility of the sources of various elements/components in the respective solutions and in mixed liquid I can be appropriately changed as needed by adjusting the concentrations. However, the applicants have surprisingly found that the addition of a dispersant during the formation of mixed liquid I in step (1) is particularly advantageous for the formation of the desired catalyst of the present invention. Without being bound by any known theory, it is believed that the addition of the dispersant in step (1) is particularly advantageous for the implementation of the various steps subsequent to step (1) in the method of the invention, so as to successfully obtain the catalyst required by the invention. Accordingly, in a preferred technical solution of the present invention, the dispersant in step (1) is at least one selected from citric acid, acetic acid, oxalic acid, urea, ammonia water and ethanolamine; and/or, the addition amount of the dispersant in the step (1) is 0.01 to 0.2%, preferably 0.01 to 0.15%, of the total weight of the catalyst.

As mentioned above, the present invention is particularly advantageous for forming the desired catalyst according to the present invention by using specific treatment conditions. Wherein, for example, the heat treatment conditions of the mixed liquid I in the step (1) are as follows: performing heat treatment at 80-150 °C, and maintaining at constant temperature for 10-30 min; preferably, the heating rate of the heat treatment is 10-20 °C/min.

In a preferable technical solution of the invention, the source of the carrier of the step (2) is a silica sol, wherein the silica sol has a solid content of 20wt%-50wt% calculated as silica, and has an average particle size of 10-35 nm.

As mentioned above, the present invention is particularly advantageous for forming the desired catalyst according to the present invention by using specific treatment conditions. Wherein, for example, in a preferred technical solution of the invention, the temperature of the heat treatment for the slurry formed in the step (6) is 60-150 °C, and the temperature is maintained constant for 5-20 min; preferably, the heating rate of the heat treatment is 8-25 °C/min.

In a preferred technical solution of the invention, various solutions are mixed while maintaining the temperatures at which they dissolve completely in steps of the method of the invention. Accordingly, for example, after the heat treatment of the mixed liquid I in the step (1), it is cooled to room temperature and then mixed with the mixed liquid II in the step (4).

In a preferred technical solution of the present invention, the drying in the step (6) is performed by spray drying, and the conditions thereof include, for example: the drying temperature is 250-350 °C, preferably 300-350 °C; and/or the drying time is 0.1-2.0h, preferably 0.2-1.0h; and/or the spray droplets have an average diameter of from 20 to 200µm, preferably from 40 to 180µm. The drying heat source for spray drying is a drying heat source commonly used in the prior art, such as air.

In a preferred technical solution of the present invention, the calcining conditions in the step (7) include: the calcining temperature is 250-700 °C; the calcining time is 30-300 min; preferably, the temperature is raised to 250-400 °C at a calcining temperature-raising rate of 5-20 °C/min, and the temperature is kept constant for 10-60 min, and then the temperature is further raised to 400-700 °C at a calcining temperature-raising rate of 5-20 °C/min, and the temperature is kept constant for 20-90 min. The calcination atmosphere is an atmosphere which is common in the prior art for calcination in catalyst preparation, such as air.

Another object of the present invention is to provide an use of the catalyst, for the reaction of preparation of acrylonitrile by ammoxidation of propylene, preferably, reaction conditions comprising: the mol ratio of propylene/ammonia gas/air in terms of O₂ is 1:(1.1-1.35):(1.8-2.5), the reaction temperature is 420-440 °C, the reaction pressure is 0.03-0.14MPa in terms of gauge pressure, and the weight hourly space velocity (catalyst loading) is 0.04-0.10h⁻¹.

The invention has the following advantageous effects
The catalyst of the invention has a more stable active phase, which is beneficial to maintain more stable reaction performance in the reaction process. The catalyst of the invention can have a high propylene conversion when used in the reaction of preparing acrylonitrile by ammoxidation of propylene.

### Examples

The present invention will now be described in detail with reference to specific examples, and it is necessary to note here that the examples are only used for further illustration of the present invention and should not be construed as limiting the scope of protection of the present invention. Non-essential improvements and adjustments to the present invention made by those skilled in the art based on the contents of present invention are still within the scope of protection of the present invention.

Furthermore, it should be noted that the various specific technical features described in the following specific embodiments can be combined in any suitable manner without contradiction. To avoid unnecessary repetition, the present invention will not describe all possible combinations separately.

Furthermore, various different embodiments of the present invention can also be arbitrarily combined, as long as they do not violate the spirit of the present invention. The resulting technical solutions are part of the original disclosure of the specification and also fall within the scope of protection of the present invention.

Reagent sources: all reagents were commercially available.

In the examples, the R and Q are respectively determined as follows:
Taking 1g of catalyst, measuring the peak areas of peaks at 20 positions of 22.8°±0.2 and 25.5°±0.2 in the X-ray diffraction of the catalyst, and
the method for determining R' and Q' by pulse-reduction experiment is as follows:
   under the condition without oxygen, 1g of catalyst is added into a reactor to carry out pulse-reduction by introducing propylene and nitrogen, wherein the gas molar ratio of the propylene to the nitrogen is 0.25, the reaction temperature is 430 °C, the catalyst load (weight space velocity) is 0.18h⁻¹, and the residence time is 0.3s; with an interval of 10min, the pulse-reduction is repeated; and after implementing the pulse-reduction for 10 times, a sample after pulse-reduction experiment is obtained.

For R and Q, and R' and Q', XRD of sample is obtained by using a X-ray powder diffractometer of Bruker, Germany, Cu-K target radiation, wavelength of 0.15406nm, scanning range of 5-80° and scanning rate of 5°/min.

The compositions of the catalysts in the following Examples and Comparative examples were calculated based on the theoretical ratio of the charged amounts.

### Example 1

581.7 g of (NH₄)₆Mo₇O₂₄·4H₂O was dissolved in water, 795.5 g of Fe(NO₃)₃·9H₂O, 77.1 g of Pr(NO₃)₃·6H₂O and 42.6 g of La(NO₃)₃·6H₂O were added and mixed, and 0.5g of urea was added to form a mixed liquid I. The mixed liquid I was heat treated at 80 °C, wherein the heating rate of the heat treatment was 10 °C/min, and the temperature was kept constant for 10 min. 3.63 g of KOH, 173.2 g of Bi(NO₃)₃·5H₂O, 524.4 g of Ni(NO₃)₂·6H₂O and 137.3 g of Mg(NO₃)₂·6H₂O were added and dissolved in water to obtain a solution III. 241.7 g of (NH₄)₆Mo₇O₂₄·4H₂O was dissolved in water, 2750 g of silica sol with a weight concentration of 40% (average particle size 25 nm) was added thereto, and the mixture was stirred for 30 minutes to obtain a mixed liquid II. The mixed liquid I was added into the mixed liquid II at constant rate in 5 min and stirred to obtain a mixed liquid IV. The solution III was added into the mixed liquid IV at constant rate in 5 min to form a slurry V. The slurry V was heated to 150 °C at heating rate of 10 °C/min and the temperature was maintained constant for 15 min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 5°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

50%K_{0.15}Fe_{5.46}Ni_{5.0}Mg_{1.5}Pr_{0.5}La_{0.3}Bi_{1.0}Mo₁₃Oₓ + 50%SiO₂

Fig. 1 was the XRD pattern for measuring R and Q, and R' and Q' for Example 1, wherein the ratio (R'+Q')/(R+Q) of the peak areas R' and Q' at 20 positions near 22.8°±0.2 and 25.5°±0.2 to the peak areas R and Q of the fresh sample was 0.85. Wherein the ratio T/R of the peak areas T and R at 20 positions near 28.2°±0.2 and 22.8°±0.2 was 4.4, and the D_{Fe} was 36%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 99.3% and the reaction remained stable.

### Example 2

524.0 g of (NH₄)₆Mo₇O₂₄·4H₂O was dissolved in water, 803.6 g of Fe(NO₃)₃·9H₂O, 78.3 g of Nd(NO₃)₃·6H₂O and 42.9 g of La(NO₃)₃·6H₂O were added and mixed, and 1.0g of urea was added to form a mixed liquid I. The mixed liquid I was heat treated at 100 °C, wherein the heating rate of the heat treatment was 15 °C/min, and the temperature was kept constant for 15 min. 3.66 g of KOH, 87.5 g of Bi(NO₃)₃ ·5H₂O, 128.4 g of Mn(NO₃)₂, 524.7 g of Co(NO₃)₂·6H₂O, 138.7 g of Mg(NO₃)₂·6H₂O and 6.09 g of AgNO₃ were added and dissolved in water to obtain a solution III. 307.8 g of (NH₄)₆Mo₇O₂₄·4H₂O was dissolved in water, 2750 g of silica sol with a weight concentration of 40% (average particle size 20 nm) was added thereto, and the mixture was stirred for 30 minutes to obtain a mixed liquid II. The mixed liquid I was added into the mixed liquid II at constant rate in 6 min and stirred to obtain a mixed liquid IV. The solution III was added into the mixed liquid IV at constant rate in 10 min to form a slurry V. The slurry V was heated to 120 °C at heating rate of 10 °C/min and the temperature was maintained constant for 15 min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 10°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

50%Ag_{0.1}K_{0.15}Fe_{5.46}Co_{5.0}Mn_{1.0}Mg_{1.5}Nd_{0.5}La_{0.3}Bi_{0.5}Mo₁₃Oₓ + 50%SiO₂

According to the XRD patterns measured for R and Q, and R' and Q', wherein the ratio (R'+Q')/(R+Q) of the peak areas R' and Q' at 20 positions near 22.8°±0.2 and 25.5°±0.2 to the peak areas R and Q of the fresh sample was 0.88. Wherein the ratio T/R of the peak areas T and R at 20 positions near 28.2°±0.2 and 22.8°±0.2 was 4.1, and the D_{Fe} was 38%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 99.0% and the reaction remained stable.

### Example 3

743.5 g of (NH₄)₆Mo₇O₂₄·4H₂O was dissolved in water, 1140.1 g of Fe(NO₃)₃·9H₂O, 66.6 g of Ce(NO₃)₃·6H₂O and 101.6 g of La(NO₃)₃·6H₂O were added and mixed, 1.5g of urea was added to form mixed liquid I. The mixed liquid I was heat treated I at 120 °C, wherein the heating rate of the heat treatment was 20 °C/min, and the temperature was kept constant for 30 min. 11.32 g of RbNO₃, 124.1 g of Bi(NO₃)₃·5H₂O, 744.5 g of Co(NO₃)₂·6H₂O, 181.2 g of Ca(NO₃)₂·6H₂O, 5.17 g of Cr₂O₃ and 4.32 g of AgNO₃ were added and dissolved in water to obtain solution III. 436.6 g (NH₄)₆Mo₇O₂₄·4H₂O was dissolved in water, 1650 g of silica sol with a weight concentration of 40% (average particle size 25 nm) was added thereto, and the mixture was stirred for 30 minutes to form a mixed liquid II. The mixed liquid I was added into the mixed liquid II at constant rate in 7 min and stirred to obtain a mixed liquid IV. The solution III was added into the mixed liquid IV at constant rate in 12 min to form a slurry V. The slurry V was heated to 100 °C at heating rate of 10 °C/min and the temperature was maintained constant for 15 min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 20°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

70%Ag_{0.05}Rb_{0.15}Fe_{5.46}Co_{5.0}Cr_{0.1}Ca_{1.5}La_{0.5}Ce_{0.3}Bi_{0.5}Mo₁₃Oₓ + 30%SiO₂

According to the XRD patterns measured for R and Q, and R' and Q', wherein the ratio (R'+Q')/(R+Q) of the peak areas R' and Q' at 2θ positions near 22.8°±0.2 and 25.5°±0.2 to the peak areas R and Q of the fresh sample was 0.90. Wherein the ratio T/R of the peak areas T and R at 20 positions near 28.2°±0.2 and 22.8°±0.2 was 3.8, and the D_{Fe} was 28%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 98.5% and the reaction remained stable.

### Example 4

638.9 g of (NH₄)₆Mo₇O₂₄·4H₂O was dissolved in water, 979.7 g of Fe(NO₃)₃·9H₂O, 38.3 g of Sm(NO₃)₃·6H₂O and 52.4 g of La(NO₃)₃·6H₂O were added and mixed, 0.5g of citric acid was added to form a mixed liquid I. The mixed liquid I was heat treated at 80 °C, wherein the heating rate of the heat treatment was 10 °C/min, and the temperature was kept constant for 10 min. 9.73g of RbNO₃, 106.6g of Bi(NO₃)₃·5H₂O, 645.8g of Ni(NO₃)₂·6H₂O, 169.1g of Mg(NO₃)₂·6H₂O, 4.44g of Cr₂O₃, 11.15g of AgNO₃ were added and dissolved in water to obtain a solution III. 375.2 g (NH₄)₆Mo₇O₂₄·4H₂O was dissolved in water, 2200 g of silica sol with a weight concentration of 40% (average particle size 25 nm) were added thereto, and the mixture was stirred for 30 minutes to obtain a mixed liquid II. The mixed liquid I was added into the mixed liquid II at constant rate in 5 min and stirred to obtain mixed liquid IV. The solution III was added into the mixed liquid IV at constant rate in 8 min to form a slurry V. The slurry V was heated to 150 °C at heating rate of 20 °C/min and the temperature was maintained constant for 15 min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 20°C/min, and the temperature was kept constant for 20 min, and then the temperature was raised to 550 °C at a temperature-raising rate of 20 °C/min, and the temperature was kept constant for 45 min, and the composition of the catalyst obtained according to above steps was represented by the following formula:

60%Ag_{0.15}Rb_{0.15}Fe_{5.46}Ni_{5.0}Cr_{0.1}Mg_{1.5}Sm_{0.5}La_{0.3}Bi_{0.5}Mo₁₃Oₓ + 40%SiO₂

According to the XRD patterns measured for R and Q, and R' and Q', wherein the ratio (R'+Q')/(R+Q) of the peak areas R' and Q' at 20 positions near 22.8°±0.2 and 25.5°±0.2 to the peak areas R and Q of the fresh sample was 0.81. Wherein the ratio T/R of the peak areas T and R at 20 positions near 28.2°±0.2 and 22.8°±0.2 was 3.5, and the D_{Fe} was 29%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 98.9% and the reaction remained stable.

### Example 5

644.6 g of (NH₄)₆Mo₇O₂₄·4H₂O was dissolved in water, 988.4 g of Fe(NO₃)₃·9H₂O, 38.7 g of Sm(NO₃)₃·6H₂O and 38.5 g of Ce(NO₃)₃·6H₂O were added and mixed, and 0.5g of oxalic acid was added to form a mixed liquid I. The mixed liquid I was heat treated at 80 °C, wherein the heating rate of the heat treatment was 10 °C/min, and the temperature was kept constant for 10 min. 9.81g of RbNO₃, 107.6g of Bi(NO₃)₃·5H₂O, 651.5g of Ni(NO₃)₂·6H₂O, 170.6g of Mg(NO₃)₂·6H₂O, 4.48g of Cr₂O₃, 6.64g of In(NO₃)₃ were added and dissolved in water to obtain a solution III. 378.5 g (NH₄)₆Mo₇O₂₄·4H₂O was dissolved in water, 2200 g of silica sol with a weight concentration of 40% (average particle diameter 25 nm) were added thereto, and the mixture was stirred for 30 minutes to obtain a mixed liquid II. The mixed liquid I was added into the mixed liquid II at constant rate in 5 min and stirred to obtain mixed liquid IV. The solution III was added into the mixed liquid IV at constant rate in 8 min to form a slurry V. The slurry V was heated to 120 °C at heating rate of 20 °C/min and the temperature was maintained constant for 15 min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 20°C/min, and the temperature was kept constant for 10 min, and then the temperature was raised to 550 °C at a temperature-raising rate of 20 °C/min, and the temperature was kept constant for 45 min, and the composition of the catalyst obtained according to above steps was represented by the following formula:

60%In_{0.05}Rb_{0.15}Fe_{5.46}Ni_{5.0}Cr_{0.1}Mg_{1.5}Sm_{0.5}Ce_{0.2}Bi_{0.5}Mo₁₃Oₓ + 40%SiO₂

According to the XRD patterns measured for R and Q, and R' and Q', wherein the ratio (R'+Q')/(R+Q) of the peak areas R' and Q' at 20 positions near 22.8°±0.2 and 25.5°±0.2 to the peak areas R and Q of the fresh sample was 0.83. Wherein the ratio T/R of the peak areas T and R at 20 positions near 28.2°±0.2 and 22.8°±0.2 was 4.1, and the D_{Fe} was 38%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 98.8% and the reaction remained stable.

### Example 6

640.1 g of (NH₄)₆Mo₇O₂₄·4H₂O was dissolved in water, 981.5 g of Fe(NO₃)₃·9H₂O, 94.9 g of Pr(NO₃)₃·6H₂O and 35 g of La(NO₃)₃·6H₂O were added and mixed, and 1.0g of ammonia water (50%) was added to form a mixed liquid I. The mixed liquid I was heat treated at 80 °C, wherein the heating rate of the heat treatment was 10 °C/min, and the temperature was kept constant for 10 min. 6.5g of RbNO₃, 106.8g of Bi(NO₃)₃·5H₂O, 647g of Ni(NO₃)₂·6H₂O, 156g of Ca(NO₃)₂·6H₂O, 4.45g of CrO₃, 13.18g of In(NO₃)₃ were added and dissolved in water to obtain a solution III. 375.9 g (NH₄)₆Mo₇O₂₄·4H₂O was dissolved in water, 2200 g of silica sol with a weight concentration of 40% (average particle size 25 nm) were added thereto, and the mixture was stirred for 30 minutes to obtain a mixed liquid II. The mixed liquid I was added into the mixed liquid II at constant rate in 5 min and stirred to obtain mixed liquid IV. The solution III was added into the mixed liquid IV at constant rate in 8 min to form a slurry V. The slurry V was heated to 100 °C at heating rate of 20 °C/min and the temperature was maintained constant for 15 min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 20°C/min, and the temperature was kept constant for 20 min, and then the temperature was raised to 550 °C at a temperature-raising rate of 20 °C/min, and the temperature was kept constant for 45 min, and the composition of the catalyst obtained according to above steps was represented by the following formula:

60%In_{0.1}Rb_{0.10}Fe_{5.46}Ni_{5.0}Cr_{0.1}Ca_{1.5}Pr_{0.5}La_{0.2}Bi_{0.5}Mo₁₃Oₓ + 40%SiO₂

According to the XRD patterns measured for R and Q, and R' and Q', wherein the ratio (R'+Q')/(R+Q) of the peak areas R' and Q' at 20 positions near 22.8°±0.2 and 25.5°±0.2 to the peak areas R and Q of the fresh sample was 0.75. Wherein the ratio T/R of the peak areas T and R at 20 positions near 28.2°±0.2 and 22.8°±0.2 was 4.7, and the D_{Fe} was 32%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 99.0% and the reaction remained stable.

### Comparative example 1

795.5g of Fe(NO₃)₃·9H₂O, 77.1g of Pr(NO₃)₃·6H₂O, 42.6g of La(NO₃)₃·6H₂O, 3.63g of KOH, 173.2g of Bi(NO₃)₃·5H₂O, 524.4g of Ni(NO₃)₂·6H₂O, 137.3g of Mg(NO₃)₂·6H₂O were added into water and dissolved to obtain a solution III. 823.4 g (NH₄)₆Mo₇O₂₄·4H₂O was dissolved in water, 2750 g of silica sol with a weight concentration of 40% (average particle size 25 nm) was added thereto, and the mixture was stirred for 30 minutes to obtain a mixed liquid I. The solution III was added into the mixed liquid I to form slurry V. The slurry V was heated to 150 °C at heating rate of 10 °C/min and the temperature was maintained constant for 15min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 5°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

50%K_{0.15}Fe_{5.46}Ni_{5.0}Mg_{1.5}Pr_{0.5}La_{0.3}Bi_{1.0}Mo₁₃Oₓ + 50%SiO₂

According to the XRD patterns measured for R and Q, and R' and Q', wherein the ratio (R'+Q')/(R+Q) of the peak areas R' and Q' at 20 positions near 22.8°±0.2 and 25.5°±0.2 to the peak areas R and Q of the fresh sample was 0.60. Wherein the ratio T/R of the peak areas T and R at 20 positions near 28.2°±0.2 and 22.8°±0.2 was 6.1, and the D_{Fe} was 6%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 94.1%.

### Comparative example 2

803.6g of Fe(NO₃)₃·9H₂O, 78.3g of Nd(NO₃)₃·6H₂O, 42.9g of La(NO₃)₃·6H₂O, 3.66g of KOH, 87.5g of Bi(NO₃)₃·5H₂O, 128.4g of Mn(NO₃)₂, 524.7g of Co(NO₃)₂·6H₂O, 138.7g of Mg(NO₃)₂·6H₂O, 6.09g of AgNO₃ were added into water and dissolved to obtain a solution III. 831.8 g of (NH₄)₆Mo₇O₂₄·4H₂O was dissolved in water, 2750 g of silica sol with a weight concentration of 40% (average particle size 20 nm) was added thereto, and the mixture was stirred for 30 minutes to obtain a mixed liquid I. The solution III was added into the mixed liquid I to form slurry V. The slurry V was heated to 150 °C at heating rate of 10 °C/min and the temperature was maintained constant for 15min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 10°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

50%Ag_{0.1}K_{0.15}Fe_{5.46}Co_{5.0}Mn_{1.0}Mg_{1.5}Nd_{0.5}La_{0.3}Bi_{0.5}Mo₁₃Oₓ + 50%SiO₂

According to the XRD patterns measured for R and Q, and R' and Q', wherein the ratio (R'+Q')/(R+Q) of the peak areas R' and Q' at 2θ positions near 22.8°±0.2 and 25.5°±0.2 to the peak areas R and Q of the fresh sample was 0.62. Wherein the ratio T/R of the peak areas T and R at 20 positions near 28.2°±0.2 and 22.8°±0.2 was 5.5, and the D_{Fe} was 8%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 95.7%.

### Comparative example 3

581.7 g of (NH₄)₆Mo₇O₂₄·4H₂O was dissolved in water, 795.5 g of Fe(NO₃)₃·9H₂O, 77.1 g of Pr(NO₃)₃·6H₂O and 42.6 g of La(NO₃)₃·6H₂O were added and mixed to form a mixed liquid I. 3.63 g of KOH, 173.2 g of Bi(NO₃)₃·5H₂O, 524.4 g of Ni(NO₃)₂·6H₂O and 137.3 g of Mg(NO₃)₂·6H₂O were added and dissolved in water to obtain a solution III. 241.7 g (NH₄)₆Mo₇O₂₄·4H₂O was dissolved in water, 2750 g of silica sol with a weight concentration of 40% (average particle size 25 nm) was added thereto, and the mixture was stirred for 30 minutes to obtain a mixed liquid II. The mixed liquid I was added into the mixed liquid II and stirred to form a mixed liquid IV. The solution III was added continuously into the mixed liquid IV to form a slurry V. The slurry V was heated to 150 °C at heating rate of 10 °C/min and the temperature was maintained constant for 15min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 5°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

50%K_{0.15}Fe_{5.46}Ni_{5.0}Mg_{1.5}Pr_{0.5}La_{0.3}Bi_{1.0}Mo₁₃Oₓ + 50%SiO₂

In XRDs measured for R and Q, and R' and Q', the ratio (R'+Q')/(R+Q) of the peak areas R' and Q' at 20 positions near 22.8°±0.2 and 25.5°±0.2 to the peak areas R and Q of the fresh sample was 0.63. Wherein the ratio T/R of the peak areas T and R at 20 positions near 28.2°±0.2 and 22.8°±0.2 was 5.9, and the D_{Fe} was 5.5%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 95.4%.

### Comparative example 4

581.7 g of (NH₄)₆Mo₇O₂₄·4H₂O was dissolved in water, 795.5 g of Fe(NO₃)₃·9H₂O, 77.1 g of Pr(NO₃)₃·6H₂O and 42.6 g of La(NO₃)₃·6H₂O were added and mixed to form a mixed liquid I. 3.63 g of KOH, 173.2 g of Bi(NO₃)₃·5H₂O, 524.4 g of Ni(NO₃)₂·6H₂O and 137.3 g of Mg(NO₃)₂·6H₂O were added and dissolved in water to obtain a solution III. 241.7 g (NH₄)₆Mo₇O₂₄·4H₂O was dissolved in water, 2750 g of silica sol with a weight concentration of 40% (average particle size 25 nm) was added, and 0.5g of urea was added to form a mixed liquid II, and the mixed liquid II was heat treated at 80 °C, wherein the heating rate of the heat treatment was 10 °C/min, and the temperature was kept constant for 10 min. The mixed liquid I was added into the mixed liquid II and stirred to form a mixed liquid IV. The solution III was added continuously into the mixed liquid IV to form a slurry V. The slurry V was heated to 150 °C at heating rate of 10 °C/min and the temperature was maintained constant for 15min. The prepared slurry was subjected to microsphere formation in a spray dryer, wherein the drying temperature was 300 °C, the drying time was 0.5h, and the average diameter of spray droplets was 100 µm, so as to obtain a particulate matter. And finally, the particulate matter was calcined in an air atmosphere, wherein the temperature was raised to 300 °C at a calcining temperature-raising rate of 5°C/min, and the temperature was kept constant for 30 min, and then the temperature was raised to 550 °C at a temperature-raising rate of 20 °C/min, and the temperature was kept constant for 45 min, and the composition of the catalyst obtained according to above steps was represented by the following formula:

50%K_{0.15}Fe_{5.46}Ni_{5.0}Mg_{1.5}Pr_{0.5}La_{0.3}Bi_{1.0}Mo₁₃Oₓ + 50%SiO₂

According to the XRD patterns measured for R and Q, and R' and Q', wherein the ratio (R'+Q')/(R+Q) of the peak areas R' and Q' at 20 positions near 22.8°±0.2 and 25.5°±0.2 to the peak areas R and Q of the fresh sample was 0.64. Wherein the ratio T/R of the peak areas T and R at 20 positions near 28.2°±0.2 and 22.8°±0.2 was 6.0, and the D_{Fe} was 4.9%.

The reaction conditions for producing acrylonitrile by ammoxidation of propylene by using the above obtained catalyst were as follows: φ38 mm fluidized bed reactor, catalyst particle size 50 µm, reaction temperature: 430 °C; reaction pressure: 0.084 MPa; catalyst loading: 300 g; catalyst propylene loading (weight hourly space velocity): 0.085 h⁻¹; the raw materials ratio (mol): C₃⁼/NH₃/air = 1/1.25/2.0. After 1000 hours of operation, the reaction results were as follows: the propylene conversion was 95.3%.

**TABLE 1 Compositions and evaluation results of catalysts obtained in Examples and Comparative examples**

| No. | composition of catalyst particles | (R'+Q ')/(R+ Q) | after 4h operation of catalyst | | after 1000h operation of catalyst | |
|---|---|---|---|---|---|---|
| | | | propylen e conversi on % | acrylonit rile selectivit y % | propyle ne convers ion % | acrylonit rile selectivit y % |
| Ex. 1 | 50%K_{0.15}Fe_{5.46}Ni_{5.0}Mg_{1.5}Pr_{0.5}La_{0. 3} | 0.85 | 99.5 | 85.4 | 99.3 | 85.8 |
| | Bi_{1.0}Mo₁₃Oₓ + 50%SiO₂ | | | | | |
| Ex. 2 | 50%Ag_{0.1}K_{0.15}Fe_{5.46}Co_{5.0}Mn_{1.0}M g_{1.5} | 0.88 | 99.1 | 85.1 | 99.0 | 85.5 |
| | Nd_{0.5}La_{0.3}Bi_{0.5}Mo₁₃Oₓ + 50%SiO₂ | | | | | |
| Ex. 3 | 70%Ag_{0.05}Rb_{0.15}Fe_{5.46}Co_{5.0}Cr_{0.1}C a_{1.5} | 0.90 | 98.9 | 85.5 | 98.5 | 85.3 |
| | La_{0.5}Ce_{0.3}Bi_{0.5}Mo₁₃Oₓ + 30%SiO₂ | | | | | |
| Ex. 4 | 60%Ag_{0.15}Rb_{0.15}Fe_{5.46}Ni_{5.0}Cr_{0.1} Mg_{1.5} | 0.81 | 99.1 | 84.8 | 98.9 | 85.1 |
| | Sm_{0.5}la_{0.3}Bi_{0.5}Mo₁₃Oₓ + 40%SiO₂ | | | | | |
| Ex. 5 | 60%In_{0.05}Rb_{0.15}Fe_{5.46}Ni_{5.0}Cr_{0.1}M g_{1.5} | 0.83 | 98.9 | 85.7 | 98.8 | 85.6 |
| | Sm_{0.5}Ce_{0.2}Bi_{0.5}Mo₁₃Oₓ + 40%SiO₂ | | | | | |
| Ex. 6 | 60%In_{0.1}Rb_{0.10}Fe_{5.46}Ni_{5.0}Cr_{0.1}Ca_{1 .5} | 0.75 | 99.2 | 84.3 | 99.0 | 84.5 |
| | Pr_{0.5}La_{0.2}Bi_{0.5}Mo₁₃Oₓ + 40%SiO₂ | | | | | |
| C. Ex 1 | 50%K_{0.15}Fe_{5.46}Ni_{5.0}Mg_{1.5}Pr_{0.5}La_{0. 3} | 0.60 | 95.9 | 83.2 | 94.1 | 83.8 |
| | Bi_{1.0}Mo₁₃Oₓ + 50%SiO₂ | | | | | |
| C. Ex 2 | 50%Ag_{0.1}K_{0.15}Fe_{5.46}Co_{5.0}Mn_{1.0}M g_{1.5} | 0.62 | 96.8 | 82.1 | 95.7 | 82.2 |
| | Nd_{0.5}La_{0.3}Bi_{0.5}Mo₁₃Oₓ + 50%SiO₂ | | | | | |
| C. Ex 3 | 50%K_{0.15}Fe_{5.46}Ni_{5.0}Mg_{1.5}Pr_{0.5}La_{0. 3} | 0.63 | 96.3 | 83.5 | 95.4 | 83.7 |
| | Bi_{1.0}Mo₁₃Oₓ + 50%SiO₂ | | | | | |
| C. Ex 4 | 50%K_{0.15}Fe_{5.46}Ni_{5.0}Mg_{1.5}Pr_{0.5}La_{0. 3} | 0.64 | 96.5 | 83.6 | 95.3 | 83.6 |
| | Bi_{1.0}Mo₁₃Oₓ + 50%SiO₂ | | | | | |

| | | | | | | |
|---|---|---|---|---|---|---|
| Note: the value of X of O_{X} in the above catalyst composition is the coordination number that satisfies the oxidation states of other elements. | | | | | | |

As can be seen from the data of the catalysts after operation for 4 hours and the data of the catalysts after operation for 1000 hours, the catalysts of the present invention have a more stable active phase, and thereby are beneficial to maintain a more stable reaction performance in the reaction process. The catalysts of the present invention can have a high propylene conversion when used in the reaction of preparing acrylonitrile by ammoxidation of propylene.

The invention has been described in detail with reference to specific embodiments and illustrative examples, but the description is not intended to limit the invention. Those skilled in the art will appreciate that various equivalent substitutions, modifications or improvements may be made to the technical solutions and embodiments of the invention without departing from the spirit and scope of the invention, and all these are within the scope of the invention. The scope of the invention is defined by the appended claims.

## Claims

1. A catalyst comprising active components and a carrier; the active components comprise Mo, Bi, Fe, at least one rare earth element, at least one alkali metal element and at least one alkaline earth metal element; the catalyst has a molybdate crystal facet distribution index RQ of 0.65 to 0.98, preferably 0.70 to 0.98; wherein: $RQ = \left(R′+Q′\right) / \left(R+Q\right) ,$ wherein the R and Q are respectively determined as follows:
taking a certain amount of catalyst, and determining the peak areas of peaks at 20 positions of 22.8°±0.2 and 25.5°±0.2 in the X-ray diffraction of the catalyst, and
the R' and Q' are respectively determined as follows:
taking a certain amount of catalyst, and introducing propylene and nitrogen under the condition without oxygen, wherein the gas molar ratio of the propylene to the nitrogen being 0.25, the reaction temperature being 430 °C, the catalyst weight space velocity being 0.18h⁻¹, and the residence time being 0.3s, so that completing one pulse-reduction; repeatedly implementing the pulse-reduction with an interval of 10min; and after implementing the pulse-reduction for 10 times, determining the peak areas of peaks at 20 positions of 22.8°±0.2 and 25.5°±0.2 in the X-ray diffraction of the catalyst after 10 times of pulse-reduction.

2. The catalyst according to claim 1, **characterized in that** the catalyst exhibits a structure of Fe element enrichment in its bulk , wherein the Fe element has a Fe distribution index D_{Fe} between bulk and surface of 10-40%, wherein: ${D}_{Fe} = \left({C}_{total Fe}-{C}_{surface Fe}\right) / {C}_{total Fe} ;$
wherein the C_{total Fe} represents the total Fe content of the catalyst;
the C_{surface Fe} represents the Fe content on the catalyst surface.

3. The catalyst according to claim 1, **characterized in that** the catalyst has a molybdate phase ratio index T/R of 2 to 5, wherein T is the peak area of peak at 20 position of near 28.2 ° ± 0.2 in the X-ray diffraction of the catalyst.

4. The catalyst according to claim 1, **characterized in that** the active components have a content of 30% to 90%, preferably 50% to 70%, calculated as oxides, based on the weight of the catalyst; and the carrier has a content of 10-70%, preferably 30-50%; preferably the carrier is silica.

5. The catalyst according to claim 1, **characterized in that** the rare earth element is at least one selected from La, Ce, Pr, Nd, and Sm, preferably at least one of La, Ce, and Nd; and/or,
the alkali metal element is at least one selected from Li, Na, K, Rb and Cs, preferably K and Rb; and/or,
the alkaline earth metal element is at least one selected from Be, Mg, Ca, Sr and Ba, and preferably Mg and Ca; and/or,
the active components further comprise an element A, wherein the element A is at least one selected from W, V, Zr, P, Nb, Ni, Co, Cr, Mn, Tl, Au, Ag, Pt, Ru, Rh, Pd, Ti, Sb, In, Sn and Te, preferably at least one selected from W, Zr, P, Nb, Ni, Co, Cr, Ag, Mn and In.

6. The catalyst according to claim 5, **characterized in that**, in the active components, based on the weight of the catalyst,
the weight content of the Mo element is 15-55%, preferably 20-45%, calculated as MoO₃; and/or,
the weight content of the Bi element is 0.5%-3.5%, preferably 1.0%-3.5%, calculated as Bi₂O₃; and/or,
the weight content of the Fe element is 1%-12%, preferably 1.5%-11%, calculated as Fe₂O₃; and/or,
the weight content of the rare earth element is 1.5%-8.5%, preferably 2.5%-5.0%, calculated as the oxide of the rare earth element; and/or,
the weight content of the alkali metal element is 0.01%-0.60%, preferably 0.05%-0.55%, calculated as oxide; and/or,
the weight content of the alkaline earth metal is 0.01%-4.0%, preferably 0.5%-3.5%, calculated as oxide; and/or,
the weight content of the element A is 0.01%-15%, preferably 0.05%-14%, calculated as oxide.

7. The catalyst according to any one of claims 1 to 6, **characterized in that**, in the active components:
the Bi/Mo atomic ratio is 0.008-0.25, preferably 0.01-0.20; and/or,
the Fe/Bi atomic ratio is 1.0-12.0, preferably 1.5-11.0; and/or,
the atomic ratio of the sum of the rare earth element, the alkali metal element and the alkaline earth metal element to Mo is 0.05-0.4, preferably 0.10-0.35; and/or,
the active components further comprise an element A, and the atomic ratio of element A/Mo is 0.01 to 1.0, preferably 0.02 to 0.9.

8. A method for preparing the catalyst according to any one of claims 1-7, wherein the amounts of the active components including Mo and the carrier are determined, and comprises the following steps performed in sequence:
(1) forming solutions respectively by adding each of the entire amount of the source of Fe element, the entire amount of the source of the at least one rare earth element, and a partial amount of the source of Mo element in the active components into solvent, mixing the solutions and adding in a dispersant to form a mixed liquid I, and subjecting the mixed liquid I to a heat treatment;
(2) forming a solution by adding the remaining amount of the source of Mo element into a solvent, and mixing the solution with the entire amount of the source of the carrier to form a mixed liquid II;
(3) mixing the sources of other active components besides the active components used in steps (1) and (2) in entire amounts and adding into a solvent to form a solution III;
(4) adding the mixed liquid I into the mixed liquid II and mixing to form a mixed liquid IV;
(5) adding the solution III into the mixed liquid IV to form a slurry V;
(6) heat treating the slurry V and drying to obtain a particulate matter;
(7) optionally, calcining the particulate matter to obtain the catalyst in particle form;
wherein, the partial amount of the source of Mo element added in the step (1) accounts for 40-80% of the entire source of Mo element.

9. The method according to claim 8, **characterized in that**,
the partial amount of the source of Mo element added in the step (1) accounts for 50-75% of the entire source of Mo element; and/or,
the dispersant is at least one selected from citric acid, acetic acid, oxalic acid, urea, ammonia water and ethanolamine; and/or,
the addition amount of the dispersant is 0.01-0.2%, preferably 0.01-0.15% of the total weight of the catalyst; and/or,
the heat treatment conditions of the mixed liquid I in the step (1) are as follows: performing the heat treatment at 80-150 °C, and maintaining at constant temperature for 10-30 min; preferably, the heating rate of the heat treatment being 10-20 °C/min.

10. The method according to claim 8, **characterized in that** the temperature for the heat treatment of the slurry in the step (6) is 60-150 °C, and is maintained at constant temperature for 5-20 min; preferably, the heating rate of the heat treatment is 8-25 °C/min.

11. The method according to claim 8, **characterized in that** the source of the carrier of step (2) is a silica sol, wherein the silica sol has a solid content of 20wt%-50wt% calculated as silica, preferably having an average particle size of 10-35 nm.

12. The method according to claim 8, **characterized in that** the solutions formed by the source of each active component are respectively saturated solutions of the sources of the active components.

13. The method according to claim 8 or claim 12, **characterized in that** the source of each active component is respectively an active element-containing compound, preferably a water-soluble compound, more preferably a water-soluble salt; and/or,
the solvent of the mixed liquids and/or the solutions is water.

14. The method according to claim 8, **characterized in that** the drying of step (6) is spray drying, and the conditions include:
drying temperature being 250-350 °C; preferably 300-350 °C; and/or,
drying time being 0.1-2.0h, preferably 0.2-1.0 h; and/or,
average diameter of spray droplets being 20 to 200µm, preferably 40 to 180µm.

15. The preparing method according to claim 8, **characterized in that** the conditions of calcining in the step (7) include: the calcining temperature being 250-700 °C; the calcining time being 30-300 min; preferably, the temperature being raised to 250-400 °C at a calcining temperature-raising rate of 5-20 °C/min, and the temperature being kept constant for 10-60 min, and then the temperature being raised to 400-700 °C at a calcining temperature-raising rate of 5-20 °C/min, and the temperature being kept constant for 20-90 min.

16. Use of the catalyst according to any one of claims 1 to 7 in the reaction of preparing acrylonitrile by ammoxidation of propylene.

17. A method for preparing acrylonitrile by ammoxidation of propylene, wherein the method is carried out in the presence of the catalyst according to any one of claims 1 to 7 under the following reaction conditions: the mol ratio of propylene/ammonia/air in terms of O₂ being 1:(1.1-1.35):(1.8-2.5), the reaction temperature being 420-440 °C, the reaction pressure being 0.03-0.14MPa in terms of gauge pressure, and the weight hourly space velocity being 0.04-0.10h⁻¹.
